**Europäisches Patentamt**

**European Patent Office**

**Office européen des brevets**

(19)

(11) Publication number : **0 507 722 A1**

## (12) EUROPEAN PATENT APPLICATION

(21) Application number : **92610018.1**

(22) Date of filing : **20.03.92**

(51) Int. Cl.⁵ : **A61M 1/16**

(30) Priority : **03.04.91 DK 586/91**

(43) Date of publication of application :
**07.10.92 Bulletin 92/41**

(84) Designated Contracting States :
**AT CH DE ES FR GB IT LI NL SE**

(71) Applicant : **POLYSTAN HOLDING A/S**
**8 Walgerholm**
**DK-3500 Vaerlose (DK)**

(72) Inventor : **Haveland, Sverre Moe**
**6B Svanholmsvej**
**DK-1905 Frederiksberg C (DK)**

(74) Representative : **Schoenning, Soeren et al**
**Internationalt Patent-Bureau Kontor for**
**Industriel Eneret, Hoeje Taastrup Boulevard**
**23**
**DK-2630 Taastrup (DK)**

(54) **Hollow fiber blood oxygenator incorporating a heat exchanger.**

(57) An oxygenator provided with a heat exchanger, said oxygenator having an annular interspace (3) between an inner tube (1) and an outer tubular shell (2), which interspace is essentially filled by a web of hollow fibres wound around the inner tube, said web extending essentially in the axial direction of the annular interspace, the end portions of the fibres being fixed in an embedment (4),(5), which closes the annular interspace at its ends, and where at the ends of the annular interspace an inlet (ZI) for blood to be oxygenated and an outlet (13) for oxygenated blood are provided, and in which the inner tube and the outer shell are connected at both ends of the hollow fibres by means of end covers (8),(9) with an annular groove, (10),(11), said groove being at one end cover provided with an inlet (12) for oxygen and at the other with an outlet (13) for the oxygen. To attain a compact construction of the oxygenator and to get a small constructional height of an oxygenator combined with a heat exchanger, the oxygenator is characteristic in that the inner tube is provided with a closed cover (16) at one end, said end being provided with openings (14) to allow the blood to flow from the inner tube to the interspace between the inner tube and the outer tubular shell, and in that the inlet for blood is positioned in the other end of the inner tube and that a helically coiled ribbed tube (17) for heat exchange is placed in the inner tube, which at its other end is provided with a cover (20) comprising connecting pieces (18) for inlet and outlet to the ribbed tube.

The present invention relates to an oxygenator provided with a heat exchanger, said oxygenator having an annular interspace between an inner tube and an outer tubular shell, which interspace is essentially filled by a web of hollow fibres wound around the inner tube, said web extending essentially in the axial direction of the annular interspace, the end portions of which are fixed in an embedment, which closes the annular interspace at its ends, and where, at the ends of the annular interspace, an inlet for blood to be oxygenated and an outlet for oxygenated blood are provided, and in which the inner tube and the outer shell are connected at both ends of the hollow fibres by means of end covers with an annular groove, said groove being at one end cover provided with an inlet for oxygen and at the other with an outlet for the oxygen.

Oxygenators are used in heart surgery, where the function of the lung system of the patient is put out of action during the operation. During surgery the patient is cooled down by means of a cooling of the patient's blood. Consequently, a heat exchanger will always be provided in the blood circulation outside the patient, by means of which the patient's blood may be given a certain temperature before being oxygenated. The heat exchanger constitutes a separate element, which is connected with the oxygenator by means of tubes, but oxygenators combined with a heat exchanger are known too. With respect to the known one-unit oxygenators the heat exchanger is either placed in continuation of the oxygenator or placed beside the oxygenator as a part of the outer shell. These solutions entail either a big constructional height of the combined unit or entail that the oxygenator is built up from many individual parts with a correspondingly big number of assemblies. It should in this connection be remembered that oxygenators are constructed to be used only once, as they cannot be sterilized and re-used, and the production price is therefore of considerable importance for how widespread the use of a certain product will be. Like to any other surgical equipment requirements are made to the reliability of the product, for which reason it should be constructed in such a way that any production errors cannot cause the failure of an operation.

The object of the present invention is to provide an oxygenator of the above kind and with a built in heat exchanger, which oxygenator is outstanding on account of its compact construction with a low construction height and the few parts, of which it consists, said parts being essentially glued or welded, while any other kind of sealing is limited as far as possible.

This object is met by the oxygenator according to the present Invention, which oxygenator is characteristic by the subject matter of the characterizing clause of claim 1.

By placing the heat exchanger in the inner tube of the oxygenator, a compact construction and a low constructional height is achieved. The inner tube is provided with a closure at one end, a chamber being thus created, which chamber is for the major part taken up by the heating element of the heat exchanger, which by being a helically coiled ribbed tube gets an extensive surface and heat transmission. The inlet and outlet of the ribbed tube are placed in a cover, which is inserted in the inner tube. By ribbed tube is in this connection to be understood thin-walled tubes with cylindrical or helical corrugations.

The blood flow takes a rational path, as the inner tube is provided with openings, through which the tempered blood may pass from the inner tube into the interspace, where the hollow fibres are placed. The rational path of the blood flow contributes to keeping the volume, which is to be filled with blood before the oxygenator is ready for use, at a minimum.

According to a preferred embodiment of the invention the cover of the inner tube is integral with the end cover for the interspace between the inner tube and the outer shell. This construction reduces the number of components in the oxygenator, whereby the production becomes more rational, and the risk of leakage into the blood flow is eliminated, which consequently reducese the risk for the patient.

Preferably the inner tube is closed by means of an element integral with the inner tube. As the inlet and the outlet to the heat exchanger are found in a cover at the other end of the inner tube, there is no need for admission through the cover, which therefore, in order to reduce the number of components, may be integral with the inner tube.

The helically coiled heat exchanger may advantageously be constructed as stated in claim 4. In this embodiment, the ribbed tube itself is used as a mandrel in the winding of the helical shape, and at the same time an effective filling up of the volume of the inner tube by the heat exchanger is achieved, the volume to be filled before use being thus minimized.

To achieve a sealing between the heat exchanger and the cover with the connecting piece, the cover is provided with two annular grooves, in which the ends of the ribbed tube are placed. Preferably the ends of the ribbed tube are embedded in an additional embedment, which contributes to an even flow path and which provides an improved sealing around the ends of the ribbed tube at the cover.

The connection between the inner tube and the cover of the inner tube is in the shape of an extension extending into the inner tube and having on the outside a cylindrical groove with an O-ring.

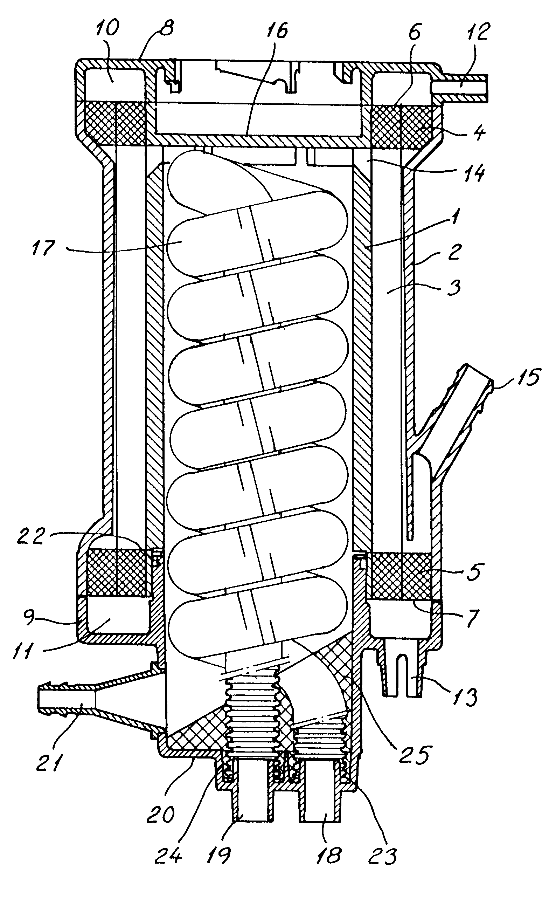

The invention will be described in detail in the following with reference to the accompanying drawing, the only figure of which is a longitudinal, sectional view of an embodiment of the oxygenator according to the invention, in which view the heat exchanger is shown schematically.

The oxygenator according to the invention comprises an inner tube 1 and an outer, tubular shell 2, which is coaxial with the inner tube, an annular interspace being created between the aforementioned tube 1 and tubular shell 2. Around the inner tube a web of hollow fibres is wound, said web being vowen together by means of warp ends running in the longitudinal direction of the web. The hollow fibres may consist of hydrofobic, microporous polypropylene hollow fibres with a wall thickness of 50 micron. Such hollow fibres are sold for instance by Akzo, Wuppertal, BRD. The wound web of hollow fibres forms an annular bundle of fibres 3, in which the individual fibres run essentially parallel with the axis of the inner tube 1. It may be advantageous, in order to get improved flow conditions in the interspace between the Individual fibres of the bundle 3, to wind it from two webs placed on top of each other, in which webs the fibres form an angle with one another and where the fibres, instead of running parallel to the axis of the inner tube run helically around the axis with a comparatively high pitch. At the ends of the inner tube and the outer shell 2 the fibres are fixed by means of an embedment 4,5, which at the same time closes the interspace placed between the inner tube and the outer shell at its ends. The casting is made by means of a two-component polyurethane moulding material. After the embedment, the outer layer of the embedment is cut off, whereby the fibre ends are opened, and whereby plane end surfaces of the fibre bundle 3 are formed, the end surfaces levelling with the ends of the inner tube 1 and the outer shell 2. On the plane surfaces 6,7 end covers 8,9 are placed, said covers each containing an annular groove 10,11, respectively. The end cover 8 is provided with a connecting piece 12 for inlet of oxygen, and the end cover 9 is provided with a corresponding connecting piece 13 for outlet of the oxygen, which has been introduced into the annular groove in the cover 8 and which has passed through the fibres in the bundle 3, having entered through the fibres opened at the cut edge 6 and passed through the fibres into the annular groove 11 in the cover 9. The connecting piece for outlet is made in such a way that an excess pressure above atmospheric cannot occur in the oxygen in the chamber formed by the annular groove.

The arrangement described does not differ essentially from the arrangement in conventional oxygenators of the hollow fibre type, and the winding and the embedment around the fibres may be made in the same way as in the common oxygenators. However, the oxygenator according to the invention differs from the known oxygenators in that the introduction of the blood to be oxygenated takes place through openings 14 in the wall of the inner tube 1. The oxygenated blood is removed in a conventional way through a connection piece 15, which is placed on an annular extension of the outer shell. The inner tube is according to the invention closed by means of a tight cover 16, which is preferably integral with the inner tube 1. Therefore, the inner tube forms a chamber closed at the top, in which chamber a heat exchanger 17 is placed. The heat exchanger 17 is according to a preferred embodiment a helically coiled, ribbed tube of corrosion-resistant material, for instance stainless stell, such as austenitic 18/9 titanium-stabilized stainless steel. The ribs are created by providing the tube with cylindrical corrugations, which partly impart considerable dimensional stability to the tube, even though it is thin-walled, and partly makes the tube flexible, so that the tube can be bent in comparatively sharp bendings without any risk of the tube collapsing. Tubes of this type are commonly available on the market and delivered for instance by United Flexible, Wales, or BOA AG, Rothenburg, Switzerland. The helical winding is made thereby that part of the tube is used as a mandrel, so that the tube so to speak is coiled around itself, the inlet 18 and the outlet 19 of the heat exchanger thus being placed adjacent to each other and in the same end of the heat exchanger. The inner tube 1 is opposite to the closure provided with a cover 20, in which the ends of the heat exchanger are embedded, and in which connecting pieces for the mounting of supply tubes to the heat exchanger are placed. The cover 20 is furthermore provided with a connection piece 21 for inlet of blood to be oxygened in the oxygenator. The cover 20 is according to the preferred embodiment according to the figure integral with the cover 9, the heat exchanger being thus mounted in the cover 20, before the combined cover 9,20 is mounted on the plane end surface 7. By the mounting the heat exchanger is introduced into the inner tube, and an extension 22 of the cover 20 extends so far into the inner tube 1, that by means of an O-ring placed in a cylindrical groove in the extension 22 a tightness is achieved between the cover 20 and the inner tube 1. After the mounting of the cover 20 the path, which the blood is to follow through the oxygenator, has been established. The blood is introduced through the connecting piece 21, conveyed upwards around the heat exchanger, passes through the openings 14 in the interspeace between the inner tube 1 and the outer shell 2, in which it is oxygened by flowing around the individual fibres in the fibre bundle 3, until it is removed at the connecting piece 15. The cover 20 is provided with two annular grooves 23,24, in which the ends of the ribbed tube are inserted. These grooves are before the insertion filled with a moulding material, which gives tightness between the ends of the tube and the cover 20, but preferably the end cover is provided with an additional embedment 25, which partly increases the tightness around the ends of the ribbed tube, partly reduces the volume, which is to be filled by blood when taking the oxygenator into use, and partly a more even flow path for the blood. The advantageous flow path is achieved thereby that the oxygenator during the hardening of the embedment is placed obliquely, so that the embedment gets an oblique surface as shown in the figure.

The position of the heat exchanger in the inner tube results in a practical exploitation of a normally not used space, but also has the effect that the combination of oxygenator and heat exchanger can be made as a compact unit with a constructional height, which does not differ essentially from the constructional height necessary for the oxygenator itself. Furthermore, a particularly good distribution of flow around the fibres in the fibre bundle is achieved, as the blood is introduced to the inner side of the annular bundle and removed at the outer side. Furthermore, it has turned out to be possible to produce the combined oxygenator of very few parts, the number of assemblies being thus reduced to a minimum.

**Claims**

1. An oxygenator provided with a heat exchanger, said oxygenator having an annular interspace between an inner tube and an outer tubular shell, which interspace is essentially filled by a web of hollow fibres wound around the inner tube, said web extending essentially in the axial direction of the annular interspace, the end portions of the fibres being fixed in an embedment, which closes the annular interspace at its ends, and where at the ends of the annular interspace an inlet for blood to be oxygenated and an outlet for oxygenated blood are provided, and in which the inner tube and the outer shell are connected at both ends of the hollow fibres by means of end covers with an annular groove, said groove being at one end cover provided with an inlet for oxygen and at the other with an outlet for the oxygen,
**characterized** in that the inner tube is provided with a closed cover at one end, said end being provided with openings (14) to allow the blood to flow from the inner tube to the interspace between the inner tube and the outer tubular shell, and in that the inlet for blood is positioned in the other end of the inner tube and that a helically coiled ribbed tube for heat exchange is placed in the inner tube, which at its other end is provided with a cover comprising connecting pieces for inlet and outlet to the ribbed tube.

2. Oxygenator according to claim 1,
**characterized** in that the cover for the inner tube is integral with the end cover of the interspace between the inner tube and the outer shell.

3. Oxygenator according to claims 1 or 2,
**characterized** in that the cover of the inner tube at one of its ends is integral with the inner tube.

4. Oxygenator according to claims 1, 2, or 3,
**characterized** in that the helically coiled ribbed tube is helically wound around a portion of the ribbed tube extending essentially along the axis of the inner tube.

5. Oxygenator according to any of the claims 1 - 4,
**characterized** in that the cover of the inner tube is provided with two annular grooves, in which the ends of the ribbed tubes are placed.

6. Oxygenator according to claim 5,
**characterized** in that the ends of the ribbed tube are enclosed in an embedment filling out at least the annular grooves.

7. Oxygenator according to any of the claims 1 - 6,
**characterized** in that the cover of the inner tube is provided with an extension extending into the inner tube and which is at its outside provided with a cylindrical groove with an O-ring.

EP 0 507 722 A1

**European Patent Office**

# EUROPEAN SEARCH REPORT

EP 92 61 0018

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 187 708 (MC NEILAB INC.)<br>* page 15, line 34 - page 16, line 26; figures 3-6 *<br>--- | 1 | A61M1/16 |
| A | US-A-4 196 075 (BENTLEY)<br>* claim 12; figures 2,3 *<br>--- | 1 | |
| A | EP-A-0 089 122 (CORDIS DOW CORP.)<br>----- | - | |
| | | | TECHNICAL FIELDS SEARCHED (Int. Cl.5 ) |
| | | | A61M |

The present search report has been drawn up for all claims

| Place of search | Date of completion of the search | Examiner |
|---|---|---|
| THE HAGUE | 06 JULY 1992 | VILLENEUVE J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

6